# EUROPEAN PATENT APPLICATION

(11) **EP 4 744 642 A1**
(43) Date of publication of application: **20.05.2026**
(21) Application number: 24843401.1
(22) Date of filing: 09.07.2024
(51) Int. Cl.: A61H 35/02, A61F 9/00

(54) **EYEBALL CLEANING DEVICE CAPABLE OF BEING EASILY USED AND MAINTAINED**

(30) Priority: 14.07.2023 KR 20230091788
(71) Applicant: Ahn, Seon Jong, Gimpo-si, Gyeonggi-do 10103 (KR)
(72) Inventor: Ahn, Seon Jong, Gimpo-si, Gyeonggi-do 10103 (KR)
(74) Representative: Danubia Patent & Law Office LLC
(86) International application number: PCT/KR2024/009802
(87) International publication number: WO 2025/018682

(57) **Abstract**

Provided is an eye irrigation device that is easy to use and maintain, including washing goggles configured to be secured to a user's head and allow user's eyes to be positioned therein, stably maintain eyelids in an open state, and supply washing water to user's eyes, a washing-water container configured to store washing water and, upon application of a predetermined pressure in one direction, collapse and spray the stored washing water, and a washing main body configured such that the washing goggles are coupled to a rear portion and the washing-water container is detachably coupled to an upper portion, in which the washing main body is configured to press the coupled washing-water container so as to supply a certain amount of washing water to the washing goggles.

## Description

### BACKGROUND

### Technical Field

The present disclosure relates to an eye irrigation device that is easy to use and maintain, and more specifically, to an eye irrigation device attachable to and detachable from a washing main body, configured to press a washing-water container in which washing water is stored to supply the stored washing water to the user's eyes for washing, configured to allow replacement of the washing-water container so that even general users can use it easily and simply, and enabling hygienic maintenance.

### Background Art

Generally, the common way to alleviate the discomfort of dry eye is to use artificial tears by directly applying the artificial tears to the eye, but regularly applying the artificial tears to the eye requires purchasing the artificial tears regularly, which involves visiting an ophthalmology clinic to get a doctor's prescription, and purchasing at a pharmacy, which takes a considerable amount of time, money, and also the hassle until obtaining the artificial tears.

Accordingly, the present applicant has proposed a device for treating dry eye and enhancing vision in Korean Patent No. 10-1483402, which includes: an upper water tank that stores saline solution and that includes an upper eye wash unit into which the saline solution is injected; a lower water tank provided under the upper water tank and including a lower eye wash unit that discharges the saline solution injected through the input hole to allow the saline solution to circulate, a circulation tank provided under the lower eye wash unit to store the discharged saline solution therein, and a partitioning panel connected to the circulation tank to cause the stored saline solution to backflow so that the saline solution is supplied back to the upper water tank side; a circulation device including a driving device provided in the upper water tank to provide a predetermined power, and a rotation assembly that receives a rotational force from the driving device to cause a forced circulation of the saline solution stored in the circulation tank by the backflow; a display device that outputs an eye muscle exercise program to enable a user to perform an eye muscle exercise; and a control device that controls driving of the circulation device and the display device and controls so that the circulation and discharge of the saline solution are performed.

However, in the case of the related art document mentioned above, because the device is configured such that the water used for washing the eye is recirculated and reused, there is a problem in that hygienic management is difficult.

In addition, since the device is configured such that the eye is washed while the water is stored in the water tank, it only provides a constant flow of water, making it difficult to provide varied washing methods suited to the user's age or ocular condition.

In particular, there are problems in that the related art document mentioned above can only be used in limited places such as specialized hospitals where disinfection can be easily performed, and because disinfection is complicated and difficult for general users to perform, there is a risk of infection due to the inability to carry out proper sterilization.

In addition, due to the high manufacturing cost, it is inevitably burdensome for general users to purchase and use, resulting in poor marketability.

### SUMMARY

In order to solve the problems described above, an object of the present disclosure is to provide an eye irrigation device that is attachable to and detachable from a washing main body, that presses a washing-water container in which washing water is stored to supply the stored washing water to the user's eyes for washing, that is configured to allow replacement of the washing-water container so that even general users can use it easily and simply, and that allows hygienic maintenance.

In addition, another object of the present disclosure is to provide an eye irrigation device that is capable of spraying washing water in various forms according to the user's age and ocular condition, thereby enabling appropriate eye irrigation tailored to the user's condition.

In addition, yet another object of the present disclosure is to provide an eye irrigation device that, by enabling the washing-water container to be used as a disposable component, allows hygienic use without performing separate cleaning or disinfection processes, and that also improves convenience of use and ease of management.

In addition, yet another object of the present disclosure is to provide an eye irrigation device which can be manufactured in a simple structure to reduce manufacturing costs, and in which only the washing-water container can be replaced and used, thereby enabling easy use by general users.

According to one aspect provided to achieve the above and other objects, an eye irrigation device that is easy to use and maintain may include: washing goggles 300 configured to be secured to a user's head and allow user's eyes to be positioned therein, stably maintain eyelids in an open state, and supply washing water to user's eyes; a washing-water container 200 configured to store washing water and, upon application of a predetermined pressure in one direction, collapse and spray the stored washing water; and a washing main body 100 configured such that the washing goggles 300 are coupled to a rear portion and the washing-water container 200 is detachably coupled to an upper portion, in which the washing main body 100 is configured to press the coupled washing-water container 200 so as to supply a certain amount of washing water to the washing goggles 300.

According to one aspect, the washing main body 100 may include: a container attachment and detachment unit 110 having an open upper portion, being configured to allow detachable coupling of the washing-water container 200, and supporting so that the washing-water container 200 and the washing goggles 300 are connected to each other; a pressing panel 130 movably coupled to a bottom surface of the container attachment and detachment unit 110 so as to be moved forward and backward, and configured to press the washing-water container 200; pressing guiding grooves 120 formed on a bottom surface of the container attachment and detachment unit 110 and configured to guide a sliding movement of the pressing panel 130; a pressing actuator 140 configured to control an operation of the pressing panel 130; washing goggle connection units 150 respectively provided on both sides and configured to be connected with the washing goggles 300; and a board housing 160 in which a built-in memory configured to store a program for each mode and a built-in battery configured to supply power are accommodated.

According to one aspect, the pressing panel 130 may include: pressing support panels 132 configured on both lower sides, passed through the pressing guiding grooves 120, and configured to be slidable along the pressing guiding grooves 120; a bottom support unit 134 connected to a lower end of the pressing support panel 132 and configured to be in close contact with a bottom surface of the washing main body 100; and a fixing flange 136 connected to a lower central portion of the bottom support unit 134 and coupled to the pressing actuator 140.

According to one aspect, the pressing actuator 140 may include: a drive motor 142 configured to provide a predetermined rotational force; a motor controller 144 configured to control whether the drive motor 142 is operated; an operating screw 148 configured to be rotated in both directions when the drive motor 142 is operated; an actuation guide 145 coupled to the fixing flange 136 and movable forward and backward along a rotation direction of the operating screw 148; an operation support panel 146 configured to fix the drive motor 142 to the washing main body 100; and a rotation support flange 147 coupled to the washing main body 100 at a position opposite to the operation support panel 146 and supporting the rotation of the operating screw 148.

According to one aspect, the washing main body 100 may further include a transparent cover 170 configured to seal the upper portion of the container attachment and detachment unit 110.

According to one aspect, the washing-water container 200 may include: a container main body 210 including a bellows portion to facilitate a collapsing operation; a pressing housing 220 configured to seal a front portion of the container main body 210 and allow the container main body 210 to collapse when the washing main body 100 is pressed; a nozzle housing 230 configured to seal a rear portion of the container main body 210 and spray the washing water stored in the container main body 210 into an interior of the washing goggles 300; and a guide plate 250 seated on an upper surface of the container main body 210 and configured to prevent inadvertent collapsing operation of the container main body 210.

According to one aspect, the nozzle housing 230 may include: nozzle main bodies 232 respectively configured on both sides of a rear portion, and inserted and passed through the washing main body 100 and connected to the washing goggles 300; a first spray hole 234 formed on an upper portion of the nozzle main body 232 and having a large diameter so that the washing water can be sprayed as a water jet; a second spray hole 236 configured below the first spray hole 234 at a predetermined interval, having a diameter smaller than that of the first spray hole 234, and having a small diameter so that the washing water can be sprayed as a water mist; sealing means 240 configured to seal the first and second spray holes 234 and 236; sealing grooves 238 respectively configured at upper and lower portions of the nozzle main body 232, and configured to guide an adhesive placement position of the sealing means 240; an inlet portion through which the washing water flows in; and an injection cap 260 for opening and closing the inlet portion.

According to one aspect, the guide plate 250 may include a front seating portion 252 formed at a front end portion and seated on an upper surface of the pressing housing 220, and a rear seating portion 254 formed at a rear end portion opposite to the front seating portion 252 and seated on an upper surface of the nozzle housing 230.

According to one aspect, the washing goggles 300 may include: a washing goggle main body 310 connected to the washing main body 100, and configured to be in close contact with a facial area of the user while being secured to the user's head to prevent leakage of the sprayed washing water; washing goggle mounting means 320 configured to fix the washing goggle main body 310 in close contact with the user's head; a main body connection member 330 connecting the washing goggle main body 310 and the washing main body 100; and eyelid fixing means 340 provided inside the washing goggle main body 310 and configured to maintain the user's eyelids in an open state during eye washing.

According to one aspect, the washing goggle main body 310 may include: an eye receiving portion 312 configured to be in close contact with the facial area of the user and configured to accommodate the user's eyes positioned therein; washing-water supply holes 316 configured to be positioned on the same line as the user's eyes and supply the washing water sprayed from the washing-water container 200; and drainage members 318 configured on both lower sides and drain the washing water after eye washing.

According to one aspect, the eyelid fixing means 340 may include: elastic support panels 342 coupled to the washing goggle mounting means 320; elastic panels 344 coupled above and below the elastic support panels 342, respectively, and configured to provide a predetermined elastic restoring force; upper and lower contact members 346 and 348 coupled to end portions of the elastic panels 344 and in contact with the user's eyelids; and pressing guiding portions 350 configured on the washing goggle main body 310 and configured to provide pressing positions of the upper and lower contact members 346 and 348.

According to embodiments of the present disclosure, the eye irrigation device is attachable to and detachable from the washing main body, presses the washing-water container in which the washing water is stored to supply the stored washing water to the user's eyes for washing, is configured to allow replacement of the washing-water container so that even general users can use it easily and simply, and allows hygienic maintenance.

In addition, according to embodiments of the present disclosure, it is possible to spray the washing water in various forms according to the user's age and ocular condition, thereby enabling appropriate eye irrigation tailored to the user's condition.

In addition, according to embodiments of the present disclosure, since the washing-water container can be used as a disposable component, hygienic use can be ensured without performing separate cleaning or disinfection processes, and convenience of use and ease of management can also be improved.

Furthermore, according to embodiments of the present disclosure, since it is possible to manufacture the device in a simple structure, it is possible to reduce manufacturing costs, and since it is possible to replace only the washing-water container and use the same, even the general users can easily use the device.

### BRIEF DESCRIPTION OF THE DRAWINGS

The above and other objects, features and advantages of the present disclosure will become more apparent to those of ordinary skill in the art by describing in detail exemplary aspects thereof with reference to the accompanying drawings, in which:

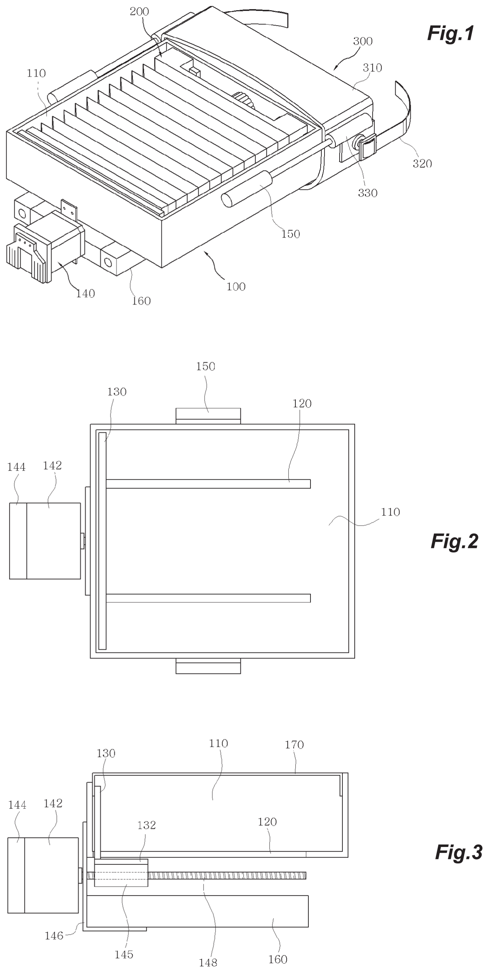

FIG. 1 is a perspective view illustrating an eye irrigation device that is easy to use and maintain according to one aspect;
FIG. 2 is a plan view illustrating a washing main body according to one aspect;
FIG. 3 is a cross-sectional view illustrating a washing main body according to one aspect;

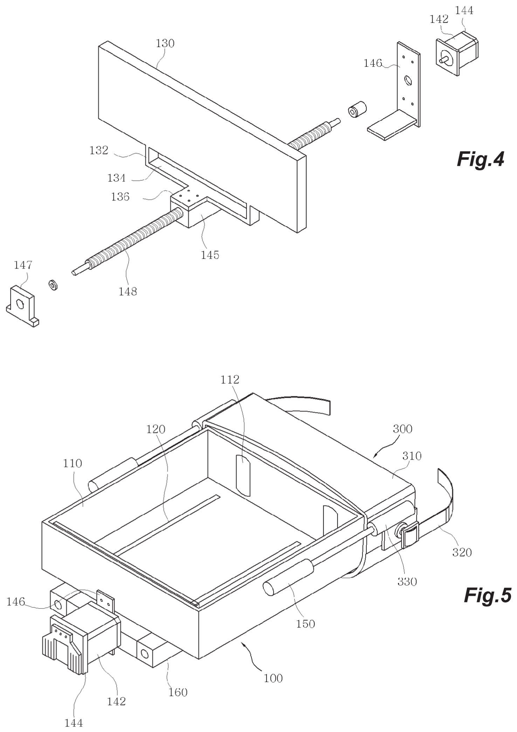

FIG. 4 is a view illustrating a pressing actuator of the washing main body according to one aspect;
FIG. 5 is a view illustrating washing goggles mounted on the washing main body according to one aspect;

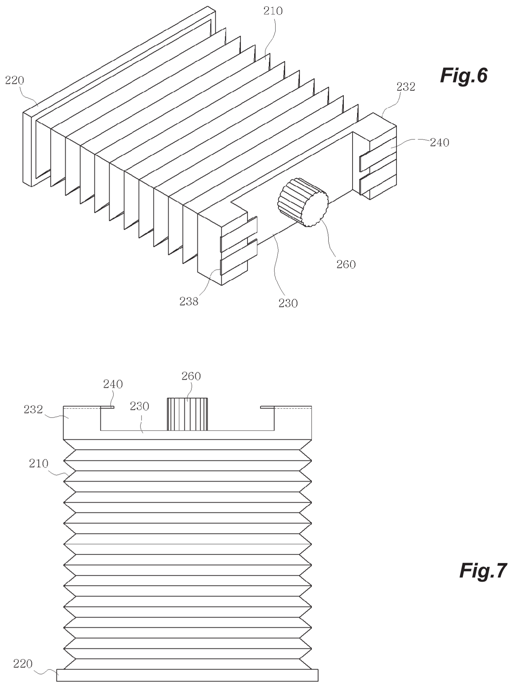

FIG. 6 is a perspective view illustrating a washing-water container according to one aspect;
FIG. 7 is a plan view illustrating the washing-water container according to one aspect;

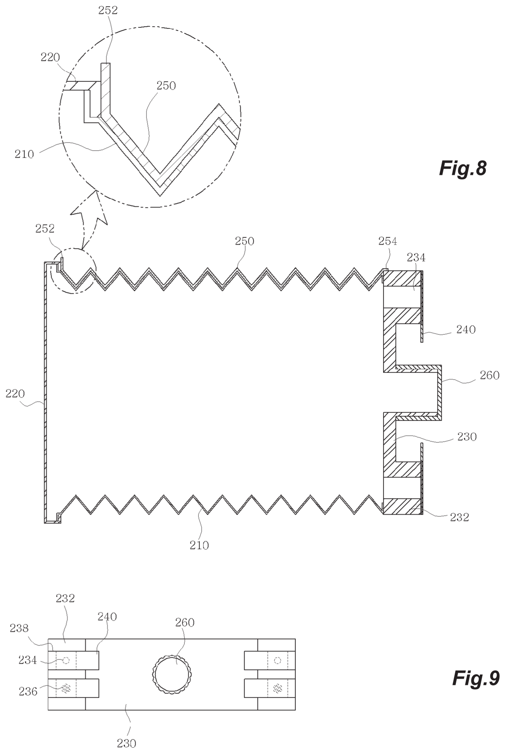

FIG. 8 is a rear view illustrating the washing-water container according to one aspect;
FIG. 9 is a cross-sectional view illustrating a main portion of the washing-water container according to one aspect;

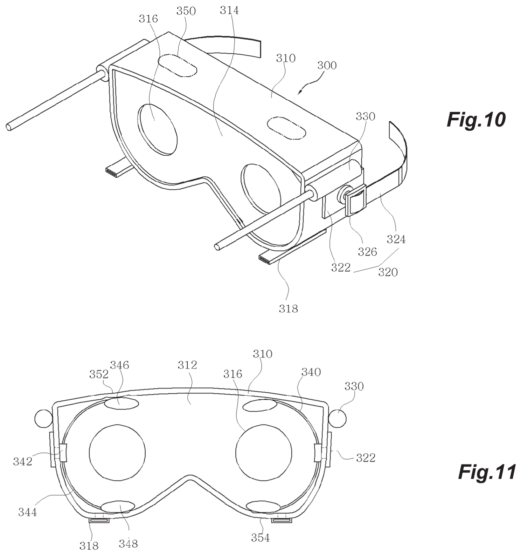

FIG. 10 is a perspective view illustrating the washing goggles according to one aspect; and
FIG. 11 is a view illustrating an eyelid fixing means of the washing goggles according to one aspect.

### DETAILED DESCRIPTION

Hereinafter, embodiments of the present disclosure will be described in detail with reference to the accompanying drawings.

As shown, an eye irrigation device that is easy to use and maintain includes: a washing main body 100 configured such that washing goggles 300 are coupled to a rear portion and a washing-water container 200 is detachably coupled to an upper portion, in which the washing main body 100 is configured to press the coupled washing-water container 200 so as to supply a certain amount of washing water to the washing goggles 300; the washing-water container 200 configured to store washing water and, upon application of a predetermined pressure in one direction, collapse and spray the stored washing water; and the washing goggles 300 configured to be secured to the user's head and allow the user's eyes to be positioned therein, and configured to stably maintain the eyelids in an open state and allow the washing water sprayed from the washing-water container 200 to be supplied to the user's eyes for washing.

The washing main body 100 includes: a container attachment and detachment unit 110 having an open upper portion, being configured to allow detachable coupling of the washing-water container 200, and supporting so that the washing-water container 200 and the washing goggles 300 are connected to each other; a pressing panel 130 movably coupled to a bottom surface of the container attachment and detachment unit 110 so as to be moved forward and backward, and configured to press the washing-water container 200; a pressing actuator 140 configured to control an operation of the pressing panel 130; washing goggle connection units 150 provided on both sides respectively and configured to be connected with the washing goggles 300; and a board housing 160 in which a built-in memory configured to store a program for each mode and a built-in battery configured to supply power are accommodated.

The container attachment and detachment unit 110 supports so that the washing goggles 300 and the washing-water container 200 are connected at a rear portion, and includes nozzle coupling units 112 configured to guide the washing water discharged from the washing-water container 200 as a stable spray toward the eyes of the user wearing the washing goggles 300.

In addition, the nozzle coupling units 112 are configured such that nozzle main bodies 232 of a nozzle housing 230 formed in the washing-water container 200 (to be described below) can be inserted therein, and the nozzle main bodies 232 can be positioned toward the washing-water supply holes 316 formed in the washing goggles 300.

In addition, elongate pressing guiding grooves 120 are formed on a bottom surface of the container attachment and detachment unit 110 along a length direction of the container attachment and detachment unit 110, and are formed at positions corresponding to each other on both sides of the bottom surface to guide the pressing panel 130 to be slid in a front-rear direction of the container attachment and detachment unit 110.

The pressing panel 130 is a component seated on the container attachment and detachment unit 110 and connected to the pressing actuator 140 to press the washing-water container 200 received in the container attachment and detachment unit 110 according to the operation of the pressing actuator 140 so that the washing water stored in the washing-water container 200 is discharged as a spray toward the washing goggles 300.

The pressing panel 130 includes: pressing support panels 132 configured on both lower sides, passed through the pressing guiding grooves 120, and configured to be slidable along the pressing guiding grooves 120; a bottom support unit 134 connected to lower ends of the pressing support panels 132 and configured to be in close contact with a bottom surface of the washing main body 100 to support a sliding movement of the pressing panels 130 stably; and a fixing flange 136 connected to a lower central portion of the bottom support unit 134 and coupled to an actuation guide 145 of the pressing actuator 140 to be described below.

In addition, the container attachment and detachment unit 110 includes the washing goggle connection units 150 which are configured on both outer sides and to which main body connection members 330 formed on the washing goggles 300 are detachably coupled.

In one example, the washing goggle connection unit 150 may be configured as a cylindrical shaft member, and may include an insertion groove of a predetermined length formed at a central portion.

In addition, the container attachment and detachment unit 110 is connected to the pressing panel 130 at a front portion, and coupled to the pressing actuator 140 that is operated to press the washing-water container 200 while sliding the pressing panel 130.

The pressing actuator 140 is coupled to the fixing flange 136 of the pressing panel 130 and is operated so that the pressing panel 130 may be moved forward and backward along the pressing guiding grooves 120 when power is applied.

The pressing actuator 140 includes: a drive motor 142 configured to provide a predetermined rotational force; a motor controller 144 configured to control whether the drive motor 142 is operated; an operating screw 148 connected to the drive motor 142 and rotated in both directions when the drive motor 142 is operated; the actuation guide 145 coupled to the fixing flange 136 and movable forward and backward along a rotation direction of the operating screw 148; an operation support panel 146 configured to fix the drive motor 142 to the washing main body 100; and a rotation support flange 147 coupled to the washing main body 100 at a position opposite to the operation support panel 146 and supporting the rotation of the operating screw 148.

In an example, the board housing 160 including the built-in memory configured to store an operation program of the operation controller 144 and the built-in battery configured to supply power to the drive motor 142 is mounted on a lower portion of the operation support panel 146.

In addition, the operation controller 144 is connected to the built-in memory of the board housing 160, and configured to control whether the drive motor 142 is rotated and also the rotational speed thereof to control a speed at which the washing-water container 200 is pressed, and a discharge amount and a discharge speed of the washing water sprayed by pressing.

The operation controller 144 may be configured with Arduino hardware, but is not limited thereto.

In an example, the memory may control whether the pressing actuator 140 is operated or not, may store a program to control a spray amount, a spray speed, and a spray pressure, may set operation condition values for each of the spray amount, the spray speed, and the spray pressure, and may store mode-specific operation programs based on the set values.

The memory may be configured to be electrically connected to the operation controller 144 described above.

Meanwhile, in one example, the washing main body 100 may further include a transparent cover 170 which is formed of a transparent material and configured to seal the open upper surface of the container attachment and detachment unit 110 to prevent entry of external foreign matter and to allow a user to visually check the pressed state of the washing-water container 200.

The washing-water container 200 is detachably inserted into the container attachment and detachment unit 110, is configured such that a rear portion is coupled to the nozzle coupling unit 112 and in communication with the washing goggles 300, is configured to collapse upon operation of the pressing panel 130 to spray the stored washing water toward the washing goggles 300, and includes a container main body 210, the nozzle housing 230, sealing means 240, and a guide plate 250.

The container main body 210 may be configured as a container having a bellows portion to facilitate a collapsing operation, and a pressing housing 220 may be coupled at a front portion and the nozzle housing 230 may be coupled at a rear portion.

The container main body 210 is formed of a synthetic resin material and may be formed in a general bellows form, but is not limited thereto.

In addition, the container main body 210 includes the guide plate 250 that is seated on the upper surface and configured to prevent inadvertent collapsing operation of the container main body 210.

The guide plate 250 may be formed in the same shape as the bellows portion of the container main body 210, may be seated in close contact, and may be formed of plastic or metal material with a predetermined rigidity.

The guide plate 250 may include a front seating portion 252 formed at a front end portion and seated on an upper surface of the pressing housing 220, and a rear seating portion 254 formed at a rear end portion opposite to the front seating portion 252 and seated on an upper surface of the nozzle housing 230.

In an example, an adhesive having a predetermined adhesive force may be applied to the front seating portion 252 and the rear seating portion 254, but is not limited thereto.

In addition, the pressing housing 220 is configured to seal the front portion of the container main body 210 while being in close contact with the pressing panel 130, and, upon sliding, the pressing panel 130 presses the container main body 210 to allow the container main body 210 to collapse.

In addition, the nozzle housing 230 is configured to spray the washing water stored in the container main body 210 into the interior of the washing goggles 300 while sealing the rear portion of the container main body 210, and includes: the nozzle main bodies 232 configured on both sides of the rear portion respectively, formed with a plurality of spray holes of different diameters, and positioned toward the washing-water supply holes 316 of the washing goggles 300 while being inserted and passed through the nozzle coupling units 112 of the container attachment and detachment unit 110; and sealing grooves 238 to which the sealing means 240 for sealing the spray holes are adhered.

In one example, the spray holes may include a first spray hole 234 formed in an upper portion of the nozzle main body 232 and having a large diameter so as to spray the washing water as a water jet, and a second spray hole 236 disposed below the first spray hole 234 at a predetermined interval, having a diameter smaller than the diameter of the first spray hole 234, and having a small diameter so as to spray the washing water as a mist.

In this case, the second spray hole 236 may include a single hole or a plurality of holes of two or more, but is not limited thereto.

In addition, the sealing grooves 238 are formed on upper and lower portions of the nozzle main body 232, respectively, and configured to guide adhesive placement positions of the sealing means 240 so that the sealing means 240 seal the first and second spray holes 234 and 236 and allow either or both of the sealed first and second spray holes 234 and 236 to open.

That is, the first and second spray holes 234 and 236 are formed in a central portion of the sealing groove 238, respectively.

The nozzle housing 230 may further include an inlet portion formed at a central portion through which the washing water flows in, and an injection cap 260 for opening and closing the inlet portion may be coupled to the inlet portion in a screw coupling manner.

The sealing means 240 is configured to prevent any inadvertent leakage of the washing water through the first and second spray holes 234 and 236, and to allow either one or both of the first and second spray holes 234 and 236 to be open so that eye washing may be performed in a mode desired by the user (water jet mode or water mist mode) during eye washing.

The sealing means 240 may be configured with a general tape, but aspects are not limited thereto, and any sealing means may be used as long as it is configured such that the user can remove the sealing means 240 from the sealing grooves 238 and then insert the washing-water container 200 into the container attachment and detachment unit 110, thus allowing the washing water stored in the washing-water container 200 to be sprayed from either one or both of the first and second spray holes 234 and 236 that are open.

The washing goggles 300 are connected to the rear portion of the container attachment and detachment unit 110 of the washing main body 100, and configured such that the nozzle main bodies 232 of the nozzle housing 230 provided in the washing-water container 200 are inserted therein and secured to the user's head thus fixing the user's eyes in position, such that the washing water from the nozzle main bodies 232 are sprayed toward the eyes for washing.

The washing goggles 300 include: a washing goggle main body 310 supported so as to be connected to the container attachment and detachment unit 110, and configured to be in close contact with a facial area of the user while being secured to the user's head to prevent leakage of the sprayed washing water; washing goggle mounting means 320 coupled to the washing goggle main body 310 and configured to fix the washing goggle main body 310 in close contact with the user's head; the main body connection members 330 configured on both sides of the washing goggle main body 310 and configured to connect the washing goggle main body 310 and the container attachment and detachment unit 110; and eyelid fixing means 340 configured inside the washing goggle main body 310 and configured to maintain the user's eyelids in an open state during eye washing.

The washing goggle main body 310 includes: an eye receiving portion 312 configured at a rear portion to be in close contact with the facial area of the user and configured to accommodate the user's eyes positioned therein; a transparent panel 314 formed of a transparent material and configured to seal a front portion of the washing goggle main body 310; and the washing-water supply holes 316 formed through the transparent panel 314, configured at a front portion to be positioned on the same line as the user's eyes, and configured to receive the nozzle main bodies 232 of the nozzle housing 230 to be inserted therein.

The washing goggle main body 310 includes drainage members 318 formed on both sides of a lower portion and configured to drain the washing water after eye washing.

The washing goggle mounting means 320 include: washing goggle mounting straps 324 adjustable according to a size of the user's head and configured to bring the washing goggle main body 310 into close contact with the facial area of the user, in which the washing goggle mounting straps 324 may be formed of a general string member having a predetermined elasticity; string adjustment clips 326 configured to adjust lengths of the washing goggle mounting straps 324; and mounting support panels 322 provided on both sides of the washing goggle main body 310 and connected to the string adjustment clips 326.

The main body connection members 330 are formed above the washing goggle mounting means 320 and configured as a general shaft member which may be inserted into and fixed to the washing goggle connection units 150 provided on the container attachment and detachment unit 110.

In this case, the main body connection member 330 may be configured as a cylinder member and may be inserted into and fixed to the washing goggle connection unit 150 as a rod portion is extended according to the control of the operation controller 144.

The eyelid fixing means 340 are provided inside the washing goggle main body 310, positioned behind the transparent panel 314, and preferably positioned above and below the washing-water supply holes 316. The eyelid fixing means 340 are configured to contact the user's eyelids and maintain the user's eyelids open by a predetermined elastic restoring force, and include elastic support panels 342, elastic members 344, and upper and lower contact members 346 and 348.

The elastic support panels 342 support the elastic operation of the elastic members 344 and support the eyelid fixing means 340 to be mounted on the washing goggle main body 310. The elastic support panels 342 are passed through both sides of the washing goggle main body 310 and fixed to the mounting support panels 322 of the washing goggle mounting means 320.

The elastic members 344 are configured as panels with a predetermined elastic restoring force, and each has one end coupled to either an upper or lower portion of the elastic support panel 342, and an opposite end coupled to either the upper or lower contact member 346 or 348.

The elastic members 344 ensure that the upper and lower contact members 346 and 348 are in close contact with upper and lower inner walls of the washing goggle main body 310, respectively, and also ensure that, during eye washing, when the user presses portions of the washing goggle main body 310 which are in close contact with the upper and lower contact members 346 and 348, the upper and lower contact members 346 and 348 are moved toward the washing-water supply holes 316 and contact the user's eyelids, and then when the user releases the pressing force applied to the washing goggle main body 310, the upper and lower contact members 346 and 348 return to their original positions, thereby maintaining the eyelids in an open state.

The upper contact members 346 are fixed to the elastic members 344 provided above the elastic support panel 342 to contact the user's upper eyelids.

In addition, the lower contact members 348 are fixed to the elastic members 344 provided below the elastic support panels 342 to contact the user's lower eyelids.

In an example, the upper contact members 346 and the lower contact members 348 may be formed of a silicon material having a predetermined adhesive force.

Meanwhile, the washing goggle main body 310 may further include pressing guiding portions 350 that guide the user so that the user is able to identify the locations of the upper and lower contact members 346 and 348 with their fingers and press the upper and lower contact members 346 and 348 more easily.

The pressing guiding portions 350 are respectively provided on upper and lower surfaces of the washing goggle main body 310 so that the user is able to easily identify the pressing positions of the upper and lower contact members 346 and 348. The pressing guiding portions 350 are provided at positions where the upper and lower contact members 346 and 348 are in contact with the washing goggle main body 310, and include an upper pressing guiding portion 352 for guiding the pressing position of the upper contact member 346 and a lower pressing guiding portion 354 for guiding the pressing position of the lower contact member 348.

## Claims

1. An eye irrigation device that is easy to use and maintain, comprising:
washing goggles configured to be secured to a user's head and allow user's eyes to be positioned therein, stably maintain eyelids in an open state, and supply washing water to user's eyes;
a washing-water container configured to store washing water and, upon application of a predetermined pressure in one direction, collapse and spray the stored washing water; and
a washing main body configured such that the washing goggles are coupled to a rear portion and the washing-water container is detachably coupled to an upper portion, wherein the washing main body is configured to press the coupled washing-water container so as to supply a certain amount of washing water to the washing goggles.

2. The eye irrigation device according to claim 1, wherein the washing main body includes:
a container attachment and detachment unit having an open upper portion, being configured to allow detachable coupling of the washing-water container, and supporting so that the washing-water container and the washing goggles are connected to each other;
a pressing panel movably coupled to a bottom surface of the container attachment and detachment unit so as to be moved forward and backward, and configured to press the washing-water container;
pressing guiding grooves formed on a bottom surface of the container attachment and detachment unit and configured to guide a sliding movement of the pressing panel;
a pressing actuator configured to control an operation of the pressing panel;
washing goggle connection units respectively provided on both sides and configured to be connected with the washing goggles; and
a board housing in which a built-in memory configured to store a program for each mode and a built-in battery configured to supply power are accommodated.

3. The eye irrigation device according to claim 2, wherein the pressing panel includes:
pressing support panels configured on both lower sides, passed through the pressing guiding grooves, and configured to be slidable along the pressing guiding grooves;
a bottom support unit connected to a lower end of the pressing support panel and configured to be in close contact with a bottom surface of the washing main body; and
a fixing flange connected to a lower central portion of the bottom support unit and coupled to the pressing actuator.

4. The eye irrigation device according to claim 3, wherein the pressing actuator includes:
a drive motor configured to provide a predetermined rotational force;
a motor controller configured to control whether the drive motor is operated;
an operating screw configured to be rotated in both directions when the drive motor is operated;
an actuation guide coupled to the fixing flange and movable forward and backward along a rotation direction of the operating screw;
an operation support panel configured to fix the drive motor to the washing main body; and
a rotation support flange coupled to the washing main body at a position opposite to the operation support panel and supporting the rotation of the operating screw.

5. The eye irrigation device according to claim 2, wherein the washing main body further includes a transparent cover configured to seal the upper portion of the container attachment and detachment unit.

6. The eye irrigation device according to claim 1, wherein the washing-water container includes:
a container main body including a bellows portion to facilitate a collapsing operation;
a pressing housing configured to seal a front portion of the container main body and allow the container main body to collapse when the washing main body is pressed;
a nozzle housing configured to seal a rear portion of the container main body and spray the washing water stored in the container main body into an interior of the washing goggles; and
a guide plate seated on an upper surface of the container main body and configured to prevent inadvertent collapsing operation of the container main body.

7. The eye irrigation device according to claim 6, wherein the nozzle housing includes:
nozzle main bodies respectively configured on both sides of a rear portion, and inserted and passed through the washing main body and connected to the washing goggles;
a first spray hole formed on an upper portion of the nozzle main body and having a large diameter so that the washing water can be sprayed as a water jet;
a second spray hole configured below the first spray hole at a predetermined interval, having a diameter smaller than that of the first spray hole, and having a small diameter so that the washing water can be sprayed as a water mist;
sealing means configured to seal the first and second spray holes;
sealing grooves respectively configured at upper and lower portions of the nozzle main body, and configured to guide an adhesive placement position of the sealing means;
an inlet portion through which the washing water flows in; and
an injection cap for opening and closing the inlet portion.

8. The eye irrigation device according to claim 6, wherein the guide plate includes:
a front seating portion formed at a front end portion and seated on an upper surface of the pressing housing; and
a rear seating portion formed at a rear end portion opposite to the front seating portion and seated on an upper surface of the nozzle housing.

9. The eye irrigation device according to claim 1, wherein the washing goggles include:
a washing goggle main body connected to the washing main body, and configured to be in close contact with a facial area of the user while being secured to the user's head to prevent leakage of the sprayed washing water;
washing goggle mounting means configured to fix the washing goggle main body in close contact with the user's head;
a main body connection member connecting the washing goggle main body and the washing main body; and
eyelid fixing means provided inside the washing goggle main body and configured to maintain the user's eyelids in an open state during eye washing.

10. The eye irrigation device according to claim 9, wherein the washing goggle main body includes:
an eye receiving portion configured to be in close contact with the facial area of the user and accommodate the user's eyes positioned therein;
washing-water supply holes configured to be positioned on the same line as the user's eyes and supply the washing water sprayed from the washing-water container; and
drainage members configured on both lower sides and drain the washing water after eye washing.

11. The eye irrigation device according to claim 9, wherein the eyelid fixing means include:
elastic support panels coupled to the washing goggle mounting means;
elastic panels coupled above and below the elastic support panels, respectively, and configured to provide a predetermined elastic restoring force;
upper and lower contact members coupled to end portions of the elastic panels and in contact with the user's eyelids; and
pressing guiding portions configured on the washing goggle main body and configured to provide pressing positions of the upper and lower contact members.
